Europäisches Patentamt

(19) European Patent Office

Office européen des brevets

(11) EP 0 919 229 A2

(12) **EUROPEAN PATENT APPLICATION**

(43) Date of publication:
02.06.1999 Bulletin 1999/22

(51) Int. Cl.⁶: $A61K\ 31/19$, $A61K\ 31/405$, $A61K\ 31/54$

(21) Application number: 98110805.3

(22) Date of filing: 06.02.1992

(84) Designated Contracting States:
AT BE CH DE DK ES FR GB GR IT LI LU NL SE

(30) Priority: 09.02.1991 EP 91101818

(62) Document number(s) of the earlier application(s) in accordance with Art. 76 EPC:
92101977.4 / 0 499 143

(71) Applicant:
B.S.D. BIO SCIENCE DEVELOPMENT SNC Di OMINI C. & ZUCCARI G.
20060 Bussero (Milano) (IT)

(72) Inventor: Bianco, Sebastiano
20143 Milano (IT)

(74) Representative:
Bianchetti, Giuseppe, Prof.
Bianchetti Bracco Minoja S.r.l.
Via Rossini, 8
20122 Milano (IT)

Remarks:
This application was filed on 12 - 06 - 1998 as a divisional application to the application mentioned under INID code 62.

(54) **Anti-reactive anti-asthmatic activity of non-steroidal anti-inflammatory drugs by inhalation**

(57) Non-steroidal anti-inflammatory drugs proved to be effective drugs for combating and preventing asthma if administered by inhalation.

Suitable pharmaceutical compositions for inhalation are aerosols with a particle size of 0.5μm to 7μm which enter the compartments of the lungs. Aqueous or aqueous-organic solutions or suspensions can be nebulized in combination with propelling agents, or mixtures of powders with microfine drugs can be administered using inhalers. These pharmaceutical compositions may comprise appropriate additives, in order to improve their relevant pharmaceutical properties.

EP 0 919 229 A2

## Description

The invention relates to the use of non-steroidal anti-inflammatory drugs for the production of an anti-reactive anti-asthmatic drug to be administered by inhalation; it relates also to non-steroidal anti-inflammatory drugs as medicaments to be inhaled for combating and preventing asthma.

Bronchial inflammation plays a key role in the current views on the pathogenetic mechanisms of bronchial asthma (Am. Rev. Respir. Dis. 1987; 136:740-51). Despite relatively large experimental evidence for the involvement of arachidonic acid metabolites in these mechanisms (J. Appl. Physiol. 1989, 66:578-583), several previous attempts to influence the bronchial responses using anti-inflammatory drugs with cyclooxygenase inhibitory activity have failed to show a clinically significant and consistent effect of these drugs in asthma (J. Allergy Clin. Immunol. 1983, 71:245-249). One of the limiting factors of the effect of these drugs on bronchial responses might be a scarce availability of these drugs in the lung following systemic administration. Indeed, a significant reduction of the bronchial response to ultrasonically nebulized water (UNW) in asthmatics after oral administration of relatively high doses of aspirin was observed (Progress in Biochemical Pharmacology (Karger, Basel) 1985, 20:132-142.2, and Allergologie, deuxieme edition, J. Charpin ed, Flammanion publ., Pris 1986, p. 683-693, and Respiration 1988, 54 (supp. 1):100-107).

Since a similar dose of the chemically related compound, sodium salicylate, which is devoid of inhibitory activity on cyclooxygenase, has no effect (L'acetilsalicilato di lisina ma non il salicilato protegge dal broncospasmo da $H_2O$. III Conferenza Italiana di Medicina Respiratoria, Milan 1990, Abstract p. 181), it can be concluded that cyclooxygenase inhibitors play a role in the therapeutic control of asthma, but relatively high doses have to be administered to afford a significant protective effect. Unfortunately, chronic treatment with such oral doses of NSAIDs causes an unacceptable rate of side effects, as indicated by two controlled studies available on the effect of treatment with high doses (1.3 to 2.6 g daily) of aspirin in asthmatics (J. Allergy. Clin. Immunol. 1984, 73:500-507; and J. Allergy Clin. Immunol. 1990, 85:59-65).

The aim of this invention was to make a breakthrough in the treatment of asthma with acceptable doses of a non-steroidal medicament.

This was accomplished by inhaling a non-steroidal anti-inflammatory drug (NSAID), preferably indomethacin, ketoprofen and tenoxicam.

Part of this invention is also a non-steroidal anti-inflammatory drug for application by inhalation in combating asthma, non-steroidal anti-inflammatory drugs of particular interest are indomethacin, ketoprofen and tenoxicam.

The studies of this invention clearly show that inhaled NSAIDs, induce a remarkable attenuation of the bronchial responses to both non-specific (UNW) and specific (immediate and late reactions to allergen) stimuli in asthmatics in the absence of side-effects.

Suitable pharmaceutical compositions for inhalation are aerosols with a particle size of from 0.5 $\mu$m to 7 $\mu$m which enter the compartments of the lungs. Aqueous or aqueous-organic solutions or suspensions which can be nebulized in combination with propelling agents, such as fluorochlorohydrocarbons, fluorinated hydrocarbons, dimethyl ether, propane, butane, nitrogen, carbon dioxide, $N_2O$, or mixtures of powders with microfine drugs, which can be administered using inhalers are particularly suitable. These pharmaceutical compositions may comprise appropriate additives, such as surface active substances, for instance phospholipids, sorbitane esters, polyoxy sorbitane esters, or oleic acids, alchols and polyols such as ethanol, glycerol, poly ethylene glycol, glucose, mannitol, sorbitol, in order to improve their relevant pharmaceutical properties.

## PATIENTS AND METHODS

**A)** Studies on non-specific bronchial hyperreactivity:

Patients:

Clinically stable patients with either allergic or non-allergic asthma with a baseline forced expiratory volume at 1 second ($FEV_1$) greater than 70% of predicted, who were free of viral or bacterial respiratory infection for at least 4 weeks were studied. None of the patients had a history of L-ASA induced asthma. All the patients were either treated with inhaled beta2-stimulants or with inhaled beta2-stimulants and topical steroids, that were withheld for 10 hours before administration of the test drug (J. Allergy Immunol. 1975; 56:323:327).

Methods:

Bronchial reactivity to ultrasonically nebulized water (UNW) was measured as previously described (Eur. J. Respir. Dis. 1980 (suppl. 106):41-9; and Pulmonary Pharmacology 1989, 1:187-191). Each subject inhaled increasing doubling doses of UNW produced by an ultrasonic nebulizer (DeVilbiss Ultra-neb 99) set to an output of 2 ml/min. The subjects were instructed to breath a tidal volume, keeping the mouthpiece between their teeth with the mouth semi-opened. Doubling doses of UNW were administered by progressively increasing the time of exposure from 30″ (1 ml) to 240″ (15 ml) and if necessary for further 240″ setting the output to 4 ml/min (31 ml). Respiratory function was monitored after each dose, until a $FEV_1$ decrease of 20% or greater compared to baseline was observed. In the cases where specific airway resistance (sRaw) was measured, this was done during normal breathing using a constant-volume body plethysmograph with a closed bag system to condition air to body temperature pressure saturation (BTPS) (Fenyves & Gut, Basel, Switzerland) before and after pretreatment, and immediately after each test. $FEV_1$, was measured using a spirometer (Vitalograph). The best value of the first 3 technically satisfactory spirograms was chosen for analysis. The dose of UNW causing a 20% $FEV_1$ decrease ($PD_{20}$) was then calculated by interpolation on the cumulative dose-response curve.

Study design:

To test the effect of NSAIDs on UNW-induced bronchoconstriction, in a group of 6 subjects with a positive response to a preliminary UNW challenge, the test was repeated twice, with intervals of 3-7 days between treatments which were either 5 ml of indomethacin 5 mg/ml for 25′ or placebo, according to a randomized protocol, by means of a nebulizer set to run to dryness in about 30′. The UNW challenge was repeated 30′ after treatment. The clinical characteristics of the patients in this group are reported in Table I.

The test was repeated a third time in three of these patients after pretreatment with a higher dose of indomethacin (5 ml at 10 mg/ml nebulized in 25′).

In four separate patients, with comparable clinical characteristics to the previous group, the effect of the two NSAIDs, ketoprofen and tenoxicam, on the immediate bronchial response tu UNW was investigated in a pilot study. This study protocol was identical to the previous study, except that indomethacin was substituted by a solution of either ketoprofen 10 mg/ml or tenoxicam 2 mg/ml. The effect of tenoxicam was tested in three, and ketoprofen in two subjects.

Additionally, in a group of patients, whose clinical characteristics are reported in Table II, the effects of tenoxicam on the immediate bronchial response to UNW was investigated in seven patients. The study protocol was identical to the previous ones except that 5 ml of a 4 mg/ml solution was nebulized over a period of 30 minutes in this test.

In a fourth group of five patients, whose clinical characteristics are reported in Table III, the effects of Ketoprofen was investigated on the immediate bronchial response to UNW. Again the same protocol was used as in the previous studies except that 7 ml of a 7.14 mg/ml solution was nebulized over a period of 40 minutes in this study. The test was performed 40 minutes after the end of nebulization.

B) Allergen specific bronchial challenge:

Patients:

Volunteers with allergic asthma who had an early obstructive response after specific allergen bronchial challenge were recruited among patients attending an allergy clinic. All the patients had a clinical history of allergic asthma and/or rhinitis, showed a positive immediate skin reaction to the clinically relevant allergen, were either asymptomatic or had very mild respiratory symptoms, had a baseline $FEV_1$ over 70% of predicted and had been free of respiratory infections for a least 4 weeks. All the patients were either untreated or under occasional topical therapy, that was withheld as described for UNW test. Patients allergic to pollen were investigated outside the pollen season.

Methods:

Specific bronchial challenge was performed as reported in N. Engl. J. Med. 1989, 321: 1069-1073.

In a preliminary bronchial challenge, the allergen (Frazioni Alfa, Dome/Hollister-Stier, Bayropharm. Italiana, Milano, Italy) was administered by a dosimeter (MEFAR, Bovezza, Italy). The apparatus was manually operated by the investigator and was set to deliver 3.7 $\mu$l/puff in 0.6 seconds, with a pause of 6 seconds between puffs.

The allergen was dissolved in normal saline at a concentration of 40 Activity Units (AU) per ml for doses up to 2.4 AU (corresponding to a delivered dose of 0.15 AU/puff ), or 160 AU/ml for doses up to 9.6 AU (0.6 AU/puff), or 320 AU/ml (1.2 AU/puff) for higher doses. AU were determined by the manufacturer using the Radio Allergy Sorbant Test (RAST) inhibition assay compared with a reference preparation characterized by skin bioreactivity. $FEV_1$ and Peak Expiratory Flow Rate (PEFR) were measured using a dry spirometer (Vitalograph, Buckingham, England) and a mini-Wright Peak Flow Meter (Clement Clarke International Ltd., London, England) before and 10 minutes after administration of a first dose of 0.15 AU. The procedure was then repeated by doubling the allergen dose until a 25% or greater decrease of $FEV_1$ from baseline was observed, or a maximum allergen dose of 19.2 AU was reached. The provocative dose of allergen causing a 25% $FEV_1$ decrease ($PD_{25}$) was then calculated by interpolation from the cumulative dose-response curve, plotted on semilogarithmic paper.

Study design:

The effect of pre-treatment with inhaled NSAID on the bronchial responses to allergen challenge was investigated in three different studies.

In a first study, the effect of pre-treatment with indomethacin (5 mg/ml) and tenoxicam (2 mg/ml) on the immediate bronchial allergic response was investigated in a double blind, cross-over study compared to placebo, using a random-number table for randomization.

Clinical characteristics of the four patients selected for the study according to the above criteria are similar to those in Table I.

Each patient performed two bronchial challenges within an interval of 4 to 14 days, using a single dose of allergen corresponding to the $PD_{25}$ determined in the preliminary challenge. Before each test, the patients either received an aerosol with 4 ml of indomethacin 5 mg/ml in saline, or 2 mg/ml of tenoxicam or a placebo (normal saline) in 15 minutes, given by means of a jet nebulizer (Mod. Soffio, Markos, Monza, Italy) set to an output of 0.27 ml/min. Immediately thereafter, the selected dose of allergen was delivered by a dosimeter. Specific airway resistance (sRaw) was measured during normal breathing using a constant-volume body plethysmograph with a closed bag system to condition air to BTPS (Fenyves & Gut, Basel, Switzerland) before and after pretreatment, and at 5, 10, 15, 20, 30, 45 and 60 minutes after the challenge. These measurements were made at least in quintuplicate and the mean was computed. $FEV_1$ was obtained by integration of flows measured with a n. 3 Fleish pneumotachograph connected to the body plethysmograph. The best value of the first 3 technically satisfactory spirograms was chosen for analysis.

Data analysis:

Data were expressed as absolute values or as a percentage of baseline values at time zero, i.e. after treatment with the active drug or placebo and immediately before challenge.

Changes of UNW reactivity were measured as doubling doses of UNW $PD_{20}$ compared to placebo, and calculated as

$$\log_2 (PD_{20} \text{ after drug}) - \log_2 (PD_{20} \text{ after placebo}).$$

An increase of the $PD_{20}$ by one doubling dose then corresponds to a 100% increase of the cumulative dose of nebulized water causing a 20% $FEV_1$ decrease.

The percentage protective effects on the specific asthmatic response for $FEV_1$ and for sRaw were calculated for each patient according to the formula:

$$((AUC \text{ placebo} - AUC \text{ treatment}) / AUC \text{ placebo}) \times 100,$$

where AUC is the area under the time-response curve of the absolute differences from baseline. All data were calculated without knowledge of the randomized treatment.

A two-way analysis of variance and a paired Student's test were used for statistical comparison of normally distributed variables, and the method of the least-significant difference was used for multiple comparisons (see Snedecor and Cochran, Statistical methods. 7th ed. Ames, Iowa University Press., 1980).

A level of p of less than 0.05 (two-tailed) was considered significant.

**RESULTS**

Studies on non-specific bronchial reactivity:

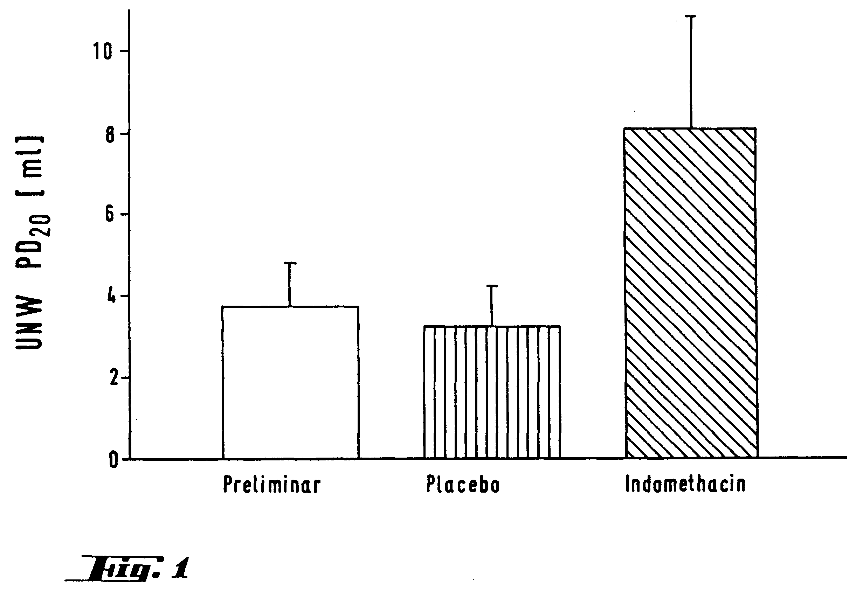

Indomethacin was generally well tolerated when given by inhalation, and was found to afford a significant protection against UNW in asthmatics. In the group of 6 patients shown in Figure 1, UNW $PD_{20}$ rose by $1.2 \pm 0,23$ doubling doses after treatment with indomethacin 5 mg/ml. In three patients treated with indomethacin 10 mg/ml the UNW $PD_{20}$ rose even higher reaching $3.14 \pm 0.23$ doubling doses (not shown).

In the pilot study both NSAIDS tenoxicam and ketoprofen were found to be suitable for administration by inhalation to patients with asthma.

The effect of tenoxicam in two patients is shown in Figures 2 and 3 in which the UNW test was conducted 20 minutes and 3 hours, respectively, after the inhalation of the drug. Similar results were obtained in a third patient. The effect of ketoprofen in one patient is also shown in Figure 4.

In the group of 7 patients tenoxicam was also well tolerated when given by inhalation. The protective effects of tenoxicam are shown in Table IV. The UNW $PD_{20}$ rose by less than 1 doubling dose resulting in a modest protection.

In the group of five patients ketoprofen was also generally well tolerated when given by inhalation. The protective effects are shown in Table V. The UNW $PD_{20}$ rose by approximately 4 doubling doses. The taste of the solution was rather unpleasant. There was also a slight irritating effect of upper airways (larynx - trachea) after the inhalation of the aerosol. The protection on the average is good although highly variable among subjects.

Studies on allergen challenge:

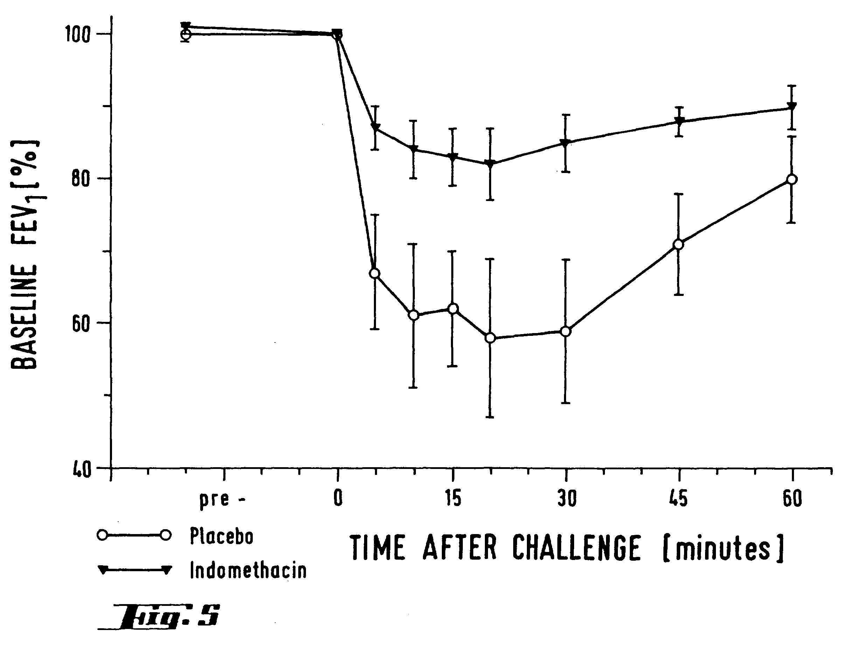

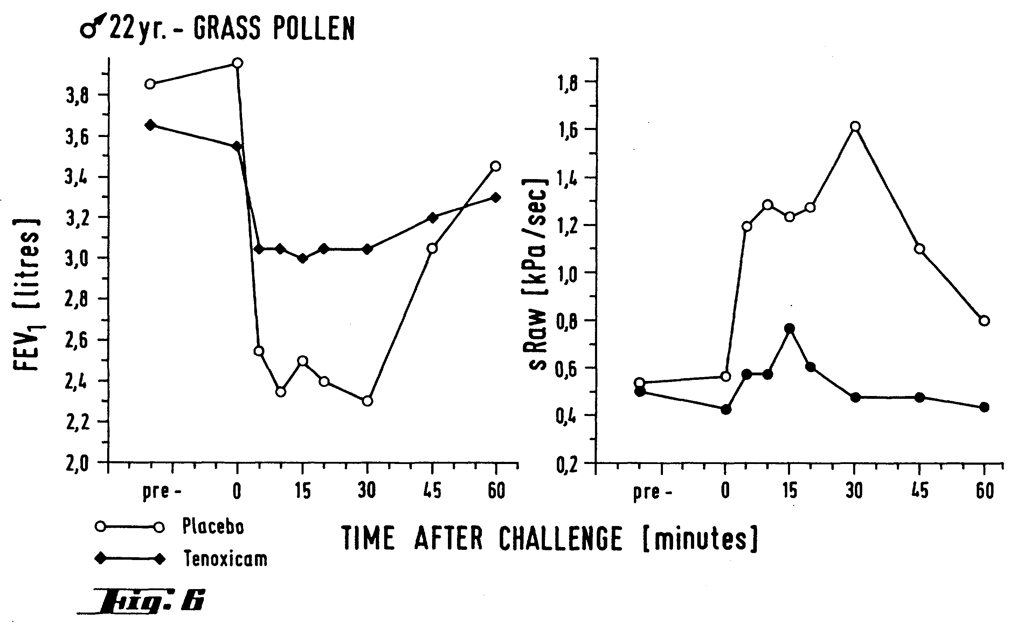

The effect of pre-treatment with inhaled indomethacin on the early asthmatic response to allergen in a group of 3 patients is reported in Figure 5. The drug provided a remarkable reduction of the intensity and the duration of the broncho-obstructive reaction. Maximum $FEV_1$ decrease in this group was $43 \pm 11\%$ after placebo and $17 \pm 5\%$ after indomethacin. The protection afforded by indomethacin, computed on $FEV_1$ differences from baseline was $51 \pm 14\%$. Also tenoxicam was effective in reducing the early asthmatic reaction in one patient as shown in Figure 6.

Discussion

These data clearly show that these problems of high oral doses, resulting in systemic side-effects, can be overcome by administration of NSAIDs by inhalation. In fact, they indicate that the inhalation of a variety of NSAIDs is able to inhibit the bronchial response to non-specific and specific bronchial stimuli in asthmatics. The inhalatory route is effective not only in reducing the side-effects in other organs which are bypassed by the topical treatment, but also in increasing the local activity of the drugs. The inhalatory route also takes advantage of a delayed drug metabolism.

It is interesting to remark that antireactive effect is exerted by NSAIDs, such as indomethacin, which affect cyclooxygenase metabolism by different mechanisms. It is particularly noteworthy that a significant inhibition of UNW- and allergen-induced responses was observed after inhalation of indomethacin, which in controlled studies has previously been shown to be ineffective on the bronchial response to UNW (J. Allergy Clin. Immunol. 1987, 79:503; and J. Allergy Clin. Immunol. 1987, 79:678-683) and to allergen challenge (J. Appl. Physiol. 1989, 66:578-583; and Rev. Respir. Dis. 1990, 1441:1445) after oral administration. Similarly, it is known that another anti-reactive drug, furosemide, is only effective on bronchial response when given by inhalation and not systemically (Pulmonary Pharmacology 1989, 1:187-191). These data thus stress the importance of the inhalatory route in revealing the therapeutic success of these drugs.

**DESCRIPTION OF THE FIGURES**

Figure 1. Effect of inhaled indomethacin (5 mg/ml) on bronchial reactivity to UNW, Clinical characteristics of the study group are reported in Table II.

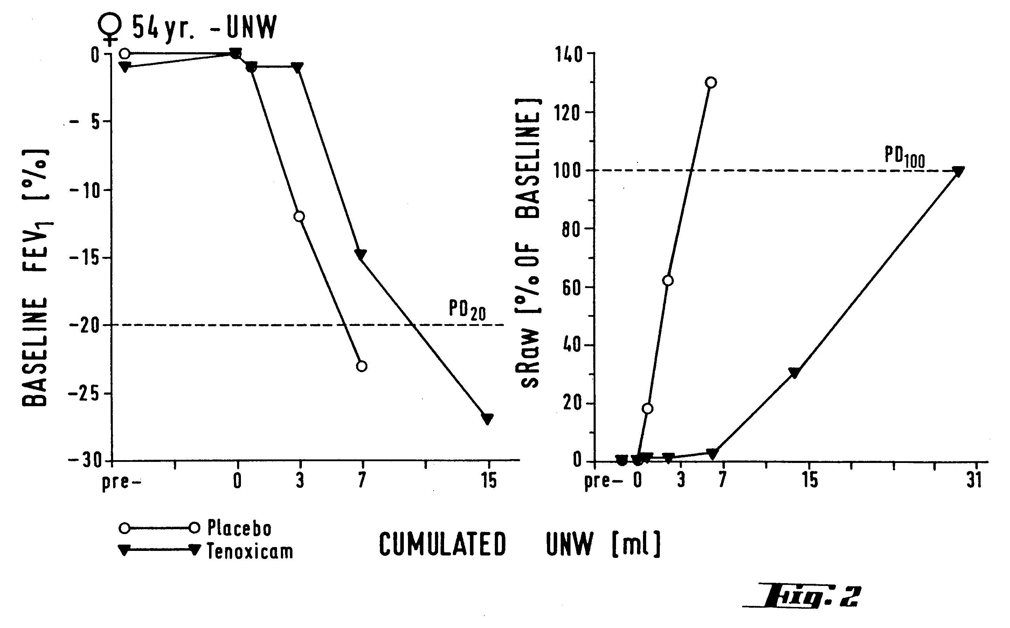

Figure 2. Effect of inhaled tenoxicam (2 mg/ml) on bronchial reactivity to UNW measured 20' after treatment in a patient with non-allergic asthma.

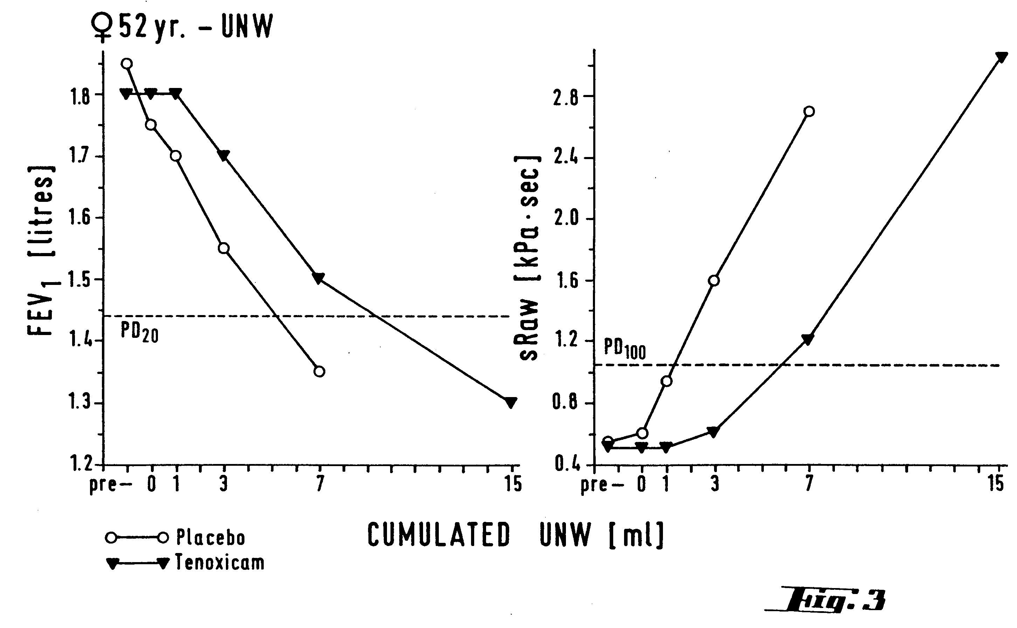

Figure 3. Effect of inhaled tenoxicam (2 mg/ml) on bronchial reactivity to UNW measured 3 hours after treatment in a patient with non-allergic asthma.

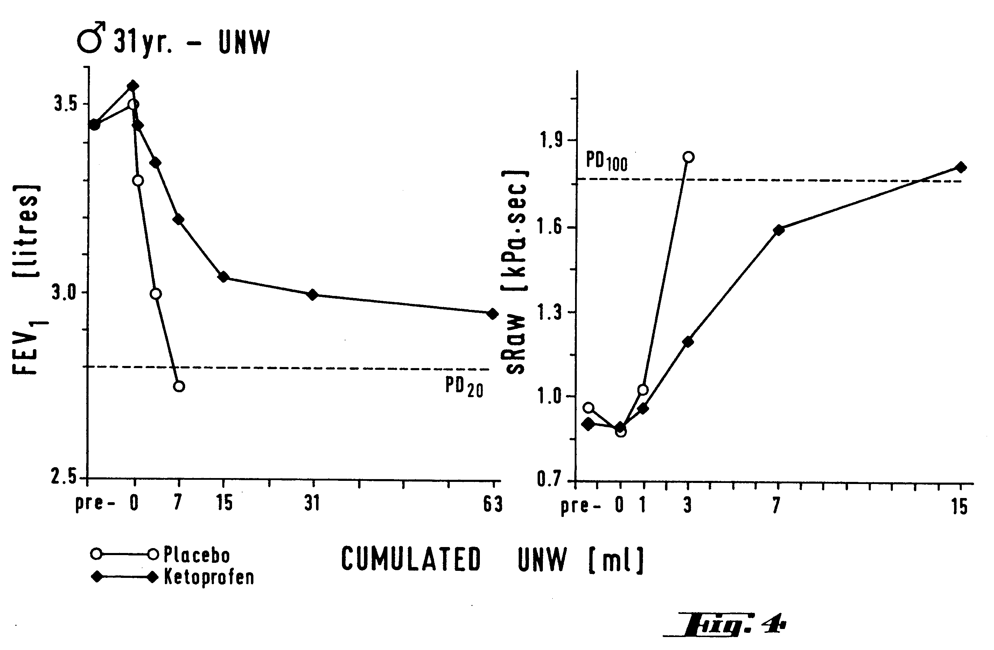

Figure 4. Effect of inhaled ketoprofen (10 mg/ml) on bronchial reactivity to UNW measured immediately after treatment in a patient with non-allergic asthma (Predicted $FEV_1$ 4.00 l).

Figure 5. Effect of inhalation of indomethacin on the early asthmatic reaction to allergen challenge in a group of three allergic patients, measured as changes of $FEV_1$. Baseline $FEV_1$ in this group was $88 \pm 3\%$ of predicted.

Figure 6. Effect of inhalation of tenoxicam on the early asthmatic reaction to allergen challenge in a patient sensitive to grass pollen ($FEV_1$ predicted 4.0 l) measured as changes of $FEV_1$ (left) and sRaw (right).

Table I

| Effect of inhaled indomethacin on UNW-induced bronchial reactions (Patients' characteristics) | | | | | | |
|---|---|---|---|---|---|---|
| N. | Sex | Age | FEV$_1$ | | ALLERGY[a] | THERAPY[b] |
| | | | litre | %pred | | |
| 1 | F | 60 | 2.12 | 87 | - | B2 + CS |
| 2 | M | 20 | 3.95 | 101 | DP | B2 |
| 3 | F | 40 | 2.15 | 84 | - | B2 |
| 4 | M | 25 | 3.33 | 85 | - | B2 + CS |
| 5 | M | 20 | 4.04 | 98 | - | B2 + CS |
| 6 | F | 18 | 2.55 | 88 | GR | B2 + CS |

a) DP: Dermatophagoides, GR: Grass pollen
b) B2: inhaled beta2-agonist, CS: inhaled steroids.

Table II

| Effect of inhaled tenoxicam on UNW-induced bronchial reactions (Patients' characteristics) | | | | | | |
|---|---|---|---|---|---|---|
| N. | Sex | Age | FEV$_1$ | | ATOPY | THERAPY[b] |
| | | | litre | %pred | | |
| 1 | M | 25 | 3.32 | 83.5 | - | B2 + CS |
| 2 | M | 43 | 3.50 | 110 | GR | B2 + CS |
| 3 | M | 18 | 5.0 | 111 | GR | B2 + CS |
| 4 | M | 35 | 3.66 | 88.5 | - | B2 + CS |
| 5 | F | 42 | 2.60 | 101 | - | B2 + CS |
| 6 | M | 19 | 4.13 | 101 | GR | B2 + CS |
| 7 | F | 60 | 3.20 | 92 | - | B2 + CS |

a) DP: Dermatophagoides, GR: Grass pollen
b) B2: inhaled beta2-agonist, CS: inhaled steroids.

Table III

| Effect of ketoprofen on UNW-induced bronchial reactions (Patients' characteristics) | | | | | | |
|---|---|---|---|---|---|---|
| N. | Sex | Age | FEV$_1$ | | ATOPY | THERAPY[b] |
| | | | litre | %pred | | |
| 1 | M | 25 | | 83.5 | - | B2 + CS |
| 2 | M | 20 | | 90 | GR | B2 + CS |
| 3 | M | 45 | | 98 | - | B2 + CS |
| 4 | F | 61 | | 90.6 | - | B2 + CS |
| 5 | M | 42 | | 114 | GR | B2 + CS |

a) DP: Dermatophagoides, GR: Grass pollen
b) B2: inhaled beta2-agonist, CS: inhaled steroids.

Table IV

| Tenoxicam UNW PD$_{20}$ (ml) | | | |
|---|---|---|---|
| | Preliminary | Placebo | Tenoxicam 5 ml - 4 mg/ml |
| 1 | 3.7 | 3.5 | 6 |
| 2 | 8.2 | 8.0 | 15 |
| 3 | 2.0 | 1.5 | 9 |
| 4 | 5.0 | 4.0 | 6 |
| 5 | 2.5 | 3.5 | 5 |
| 6 | 8.0 | 6.75 | 6.25 |
| 7 | 4.8 | 6.0 | 7.7 |
| x | 4.9 | 4.75 | 7.85 |
| SD | 2.4 | 2.25 | 3.4 |

Table V

| Ketoprofen UNW PD$_{20}$ (ml) | | | |
|---|---|---|---|
| | Preliminary | Placebo | Ketoprofen 7 ml - 7.14 mg/ml |
| 1 | 2.5 | 3.5 | 59 (PD$_{15}$) |
| 2 | 4.5 | 7.5 | 23 |
| 3 | 6.2 | 6.2 | 59 |
| 4 | 4.0 | 4.8 | 10 |
| 5 | 2.8 | 2.5 | 6 |
| x | 4.0 | 4.9 | 31.4 |
| SD | 1.48 | 2.0 | 25.9 |

**Claims**

1. Use of a non-steroidal anti-inflammatory drug for the preparation of a medicament for the inhalation therapy of asthma.

2. Use according to claim 1 wherein the non-steroidal anti-inflammatory drug is indomethacin.

3. Use according to claim 1 wherein the non-steroidal anti-inflammatory drug is ketoprofen.

4. Use according to claim 1 wherein the non-steroidal anti-inflammatory drug is tenoxicam.

_Fig. 1_

EP 0 919 229 A2

Fig. 2

Fig. 3

♂ 31yr. – UNW

FEV₁ [litres]

$FEV_1$ [litres]

sRaw [kPa·sec]

CUMULATED UNW [ml]

PD₂₀

PD₁₀₀

○ Placebo
◆ Ketoprofen

pre– 0  7  15  31  63

pre– 0 1  3  7  15

2.5  3.0  3.5

0.7  1.0  1.3  1.6  1.9

*Fig. 4*

12

Fig. 5

♂22yr. - GRASS POLLEN

FEV₁ [litres]
sRaw [kPa/sec]
TIME AFTER CHALLENGE [minutes]

○——○ Placebo
◆——◆ Tenoxicam

Fig. 6

EP 0 919 229 A2